# EUROPEAN PATENT APPLICATION

(11) **EP 4 796 106 A1**
(43) Date of publication of application: **26.08.2026**
(21) Application number: 26159219.0
(22) Date of filing: 18.02.2026
(51) Int. Cl.: A61K 9/00, A61K 9/48, A23L 33/00, A61K 8/00

(54) **CAPSULES FOR APPETITE SUPPRESSION**

(30) Priority: 20.02.2025 GB 202502471
(71) Applicant: Altmann, Eran, 3457316 Haifa (IL)
(72) Inventor: Altmann, Eran, 3457316 Haifa (IL)
(74) Representative: Pearl Cohen UK

(57) **Abstract**

A capsule for appetite suppression comprises an outer layer adapted to persist in a stomach environment and an inner volume enclosed by the outer layer. The outer layer may include a semipermeable membrane that allows interaction with stomach contents, enhancing the sensation of fullness. The capsule is designed to occupy space in the stomach, thereby reducing available volume for food intake. A kit may include multiple capsules and means for filling the inner volume with substances such as water or low-calorie liquids. The method involves swallowing an operative number of capsules to achieve the desired appetite-suppressing effect. The capsules are made from materials commonly used in slow-release drug delivery systems, ensuring safety and biocompatibility.

## Description

### FIELD OF THE INVENTION

The invention relates generally to capsules for appetite suppression, in particular to capsules adapted to occupy volume in the stomach.

### BACKGROUND

Obesity is a significant global health concern, leading to increased risk of related health conditions and comorbidities, such as diabetes, cardiovascular disease, and certain types of cancer, imposing a heavy burden on healthcare systems. Conventional approaches to managing obesity often involve surgical interventions, such as different bariatric surgeries, a gastric (e.g. laparoscopic) band, or intragastric balloon, which are invasive, can be expensive, require lengthy recovery periods, and pose associated risks. These procedures aim to decrease stomach capacity (e.g. volume) to curb hunger, but they are not appropriate for all individuals due to their invasive nature and potential complications.

On the other hand, pharmacological approaches, such as dietary aids and appetite control products, often involve the use of drugs that can have side effects and may not be suitable for long-term use. Furthermore, many appetite control products available in the market lack the desired efficacy, leading to unsatisfactory results for users seeking sustainable weight management solutions.

There is an increasing need for solutions that can effectively manage appetite and support weight reduction without resorting to surgery or medications. The challenge is to develop a method that is safe, user-friendly, and capable of providing a lasting sensation of fullness, thereby reducing food intake and aiding in weight management.

### BRIEF SUMMARY OF THE INVENTION

According to one or more embodiments of the invention, there is provided a capsule for use in appetite suppression, the capsule comprising an outer layer and an inner volume enclosed by the outer layer, wherein the outer layer is adapted to persist in an acidic stomach environment.

According to some embodiments, the inner volume is empty and the capsule comprises no pharmaceutically active ingredients.

According to some embodiments, the inner volume is comprised throughout of the same material as the outer layer, or wherein the inner volume comprises at least one of: water, a low-calorie liquid, a low-calorie powder, or an alkaline substance.

According to some embodiments, the outer layer comprises a membrane selected to be semi-permeable to stomach acid towards the inner volume.

According to some embodiments, the inner volume comprises a material that expands in volume when in contact with stomach acid.

According to some embodiments, the outer layer is biodegradable.

According to one or more embodiments there is provided a kit comprising a plurality of capsules for use in appetite suppression, each capsule comprising an outer layer and an inner volume enclosed by the outer layer, wherein the outer layer is adapted to persist in an acidic stomach environment, the kit further comprising an indication that the kit is suitable for use in appetite suppression.

According to some embodiments, the kit comprises means for filling the inner volume of the plurality of capsules.

According to some embodiments, the kit comprises a quantity of substance for filling the inner volume of the plurality of capsules.

According to some embodiments, the substance is one of: water, a low-calorie liquid, a low-calorie powder, or an alkaline substance.

According to some embodiments, the indication comprises an operative number of capsules to be swallowed, the operative number determined based on an occupying volume of each capsule in the stomach.

According to some embodiments, the operative number is greater than or equal to 5.

According to one or more embodiments there is provided a capsule for use in a method of suppressing appetite, the capsule comprising an outer layer and an inner volume enclosed by the outer layer, wherein the outer layer is adapted to persist in an acidic stomach environment, and wherein the method of suppressing appetite comprises swallowing an operative number of the capsules, wherein the operative number is determined based on an occupying volume of each capsule in the stomach.

According to some embodiments, the inner volume is empty and the capsule comprises no pharmaceutically active ingredients.

According to some embodiments, the inner volume is comprised throughout of the same material as the outer layer, or wherein the inner volume comprises at least one of: water, a low-calorie liquid, a low-calorie powder, or an alkaline substance.

According to some embodiments, the outer layer comprises a membrane selected to be semi-permeable to stomach acid towards the inner volume.

According to some embodiments, the outer layer is biodegradable.

According to some embodiments, the operative number is greater than or equal to 5.

According to one or more embodiments there is provided a pellet for use in appetite suppression, the pellet formed throughout of a material adapted to persist in an acidic stomach environment.

According to some embodiments, the material is biodegradable.

According to some embodiments, the pellet is coated in an alkaline substance.

According to one or more embodiments there is provided a kit comprising a plurality of pellets for use in appetite suppression, each pellet formed of a material adapted to persist in an acidic stomach environment, the kit further comprising an indication that the kit is suitable for use in appetite suppression.

According to some embodiments, the indication comprises an operative number of pellets to be swallowed, the operative number determined based on an occupying volume of each pellet in the stomach.

According to one or more embodiments there is provided a pellet for use in a method of suppressing appetite, the pellet formed throughout of a material adapted to persist in an acidic stomach environment, and wherein the method of suppressing appetite comprises swallowing an operative number of the pellets, wherein the operative number is determined based on an occupying volume of each pellet in the stomach.

According to some embodiments, the material is biodegradable.

According to some embodiments, the pellet is coated in an alkaline substance.

According to some embodiments, the operative number is greater than or equal to 5.

### BRIEF DESCRIPTION OF THE DRAWINGS

Non-limiting examples of embodiments of the disclosure are described below with reference to figures attached hereto. Dimensions of features shown in the figures are chosen for convenience and clarity of presentation and are not necessarily shown to scale. The subject matter regarded as the invention is particularly pointed out and distinctly claimed in the concluding portion of the specification. The invention, however, both as to organization and method of operation, together with objects, features, and advantages thereof, may be understood by reference to the following detailed description when read with the accompanied drawings. Embodiments are illustrated without limitation in the figures, in which like reference numerals indicate corresponding, analogous, or similar elements, and in which:
Fig. 1A is a schematic diagram illustrating an example structure of a capsule for appetite suppression, according to some embodiments of the invention;
Fig. 1B is a schematic diagram of a capsule designed for appetite suppression including a filled volume, according to some embodiments of the invention;
Fig. 1C is a schematic diagram illustrating the capsule's structure and an exemplary semi-permeable interaction of the capsule with stomach acid, according to some embodiments of the invention;
Fig. 2 is a schematic diagram illustrating a pellet used for appetite suppression, according to some embodiments of the invention; and
Fig. 3 is a schematic diagram of capsules or pellets occupying space in a stomach for appetite suppression, according to some embodiments of the invention;

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements.

### DETAILED DESCRIPTION

In the following detailed description, numerous specific details are set forth in order to provide a thorough understanding of the invention. However, it will be understood by those skilled in the art that the present invention can be practiced without these specific details. In other instances, well-known methods, procedures, components, modules, units and/or circuits have not been described in detail so as not to obscure the invention.

Embodiments of the invention may address the challenges identified above by providing a novel method for appetite suppression and weight management. Embodiments of the invention include the provision and/or use of capsules or pellets made from materials commonly used in slow-release (e.g. delayed release, sustained release) drug delivery systems. In some embodiments, the capsules or pellets contain no pharmaceutically active ingredients and are primarily designed to reduce stomach volume when consumed in sufficient quantities, thereby suppressing appetite without the need for invasive procedures or pharmacological interventions.

The capsules or pellets according to embodiments of the invention may be made from materials already used in the pharmaceutical industry, ensuring safety for consumption in large quantities. Because capsules/pellets according to some embodiments of the invention do not contain any pharmaceutically active ingredients, there is no risk of "over-dosing". A capsule according to some embodiments of the invention is, for example, merely a shell made of proven safe materials that occupies space in the stomach, thereby reducing available stomach volume when taken in large quantities (e.g. 10 or more). By occupying space in the stomach, the capsules create a sensation of fullness (e.g. satiety), helping individuals manage their appetite and reduce food intake. This technique offers a safe, non-invasive, and effective solution for weight management, addressing the limitations of existing methods.

As used herein, a pharmaceutically active ingredient may refer to a component in a pharmaceutical product or dietary supplement that is biologically active and responsible for the intended therapeutic effect or health benefit. A pharmaceutically active ingredient is the primary substance that produces the desired effect in the body, distinguishing it from pharmaceutically inactive ingredients, which serve as carriers or fillers without therapeutic effects.

Typically, a capsule in the context of pharmaceuticals and dietary supplements is a small, typically cylindrical or ovoid container made of gelatin or other suitable material that encloses a dose of medication, supplement, or other pharmaceutically active ingredient . Capsules are designed to be swallowed whole, allowing for the controlled release of their contents in the digestive system. They can contain a variety of pharmaceutically active ingredients, including powders, or liquids, and are often used for their ease of ingestion and ability to mask the taste of the contents.

As used herein, "capsule" may refer to a casing, shell, or otherwise encapsulating article commonly used in the pharmaceutical industry. A capsule may be a hard-shelled capsule or a soft-shelled capsule. A hard-shelled capsule may comprise two halves or portions joined at a seam and separable in a destructive or non-destructive manner. Some hard shell capsules may be referred to as being in a "locked" position when assembled. A soft-shelled capsule typically comprise a single piece which is sealed with a drop of material after filling. A capsule according to embodiments of the invention may differ from a capsule ordinarily used in the pharmaceutical industry in that it may contain no pharmaceutically active ingredients. For example, a capsule according to some embodiments of the invention may be hollow (e.g. have an inner empty volume or void).

As used herein, "pellet" may refer to a generally solid entity formed of the same material extending throughout its cross-section, without substantial voids or empty space within its volume. A pellet may be, for example, an integral whole. A pellet may have the advantage of taking longer to dissolve in the stomach and thus providing an appetite suppressing effect for longer as compared to, for example, a capsule having only an outer layer encapsulating an inner volume. A pellet may be formed of a material other than compacted powder, for example a pellet may be made of solid gelatin or solid polymer of the types of polymers used in sustained release drug delivery described herein.

Capsules and/or pellets may be referred to as a pill, tablet, and/or caplet. Capsule and pellet may be used interchangeably when the context is clear, for example when the principle is applicable irrespective of the presence of an inner encapsulated volume.

Capsules and/or pellets according to some embodiments of the invention may have any form factor. For example they may have an ovoid shape, a spherical shape, a coin shape (e.g. circular shape), a square shape (e.g. rhombus or diamond shape), or a cylindrical shape.

As used herein "persist" may refer to an ability to maintain integrity (e.g. not dissolving to an extent to expose the inner volume) over a prolonged period, for example: longer than 1 hour, longer than 5 hours, and/or longer than 24 hours. In some cases, persist may mean dissolving by less than 50% of net weight over a given time, for example 1 hour, 5 hours, or 24 hours.

As used herein "slow", for example in the context of slow delivery or slow-release capsules/pellets, may refer to a time-release formulation of the material to achieve delayed breakdown in the capsule material, which for traditional capsules increases the time scales upon which a pharmaceutically active ingredient may be released. Capsules according to some embodiments of the present invention do not contain any pharmaceutically active ingredients, such that it is not the delayed release of the pharmaceutically active ingredient which is desired, but the delayed breakdown of the capsule/pellet so that it retains an occupying volume in the stomach over a long time period (e.g. persists for longer than 1 hour, longer than 5 hours, and/or longer than 24 hours), thereby prolonging the appetite suppressing effect compared to capsules which are designed to be broken down within minutes of ingestion (e.g. immediate-release formulations).

As used herein "stomach environment" may generally be understood as relating to the highly acidic (e.g. pH 1.5-3.5) conditions within the stomach. It will be understood that gastrointestinal environment includes, but is not limited to, the stomach.

As used herein "low-calorie", for example low-calorie liquid or powder, may refer to a substance having a nutritional energy value of less than 10 kcal per unit measurement, for example having less than 10 kcal per 100g (e.g. less ≤ 5 kcal per 100g), or less than 10 kcal per 100ml (e.g. less ≤ 5 kcal per 100ml). Low-calorie may include zero calorie (e.g. 0 kcal) or substantially zero calories (e.g. less than 0.5 kcal per unit measurement).

A low-calorie liquid or powder may include a sweetener, such as an artificial or natural sweetener, for example one or more of: acesulfame K (E950), aspartame (E951), erythritol (E968), saccharin (E954), sorbitol (E420), steviol glycosides (E960), sucralose (E955), and/or xylitol (E967). Other sweeteners may be used.

Materials from which the capsules or pellets may be made may include the types of materials (such as polymers) commonly used in slow delivery capsules in the pharmaceutical industry for controlled release (e.g. delayed release, sustained release) applications. These materials can include: Gelatin; Hydroxypropyl methylcellulose (HPMC); Polyethylene glycol (PEG), also known as polyethylene oxide (PEO) or polyoxyethylene (POE); Polylactic acid (PLA); Polyglycolic acid (PGA); Poly(lactic-co-glycolic acid) (PLGA); Ethylcellulose; Cellulose acetate phthalate (CAP), also known as cellacefate (INN); Polyvinyl alcohol (PVA); Chitosan; Alginate; Eudragit ^{®} (a brand of methacrylate copolymers); Polycaprolactone (PCL); Starch-based polymers; and/or Polyvinylpyrrolidone (PVP). Other materials may be used. In some embodiments, vegetarian or vegan alternatives to gelatin may be used.

The capsule/pellet material may be selected based on the material's ability to control their stability in the stomach and/or gastrointestinal environment and their biocompatibility.

The Inventor has realised that such capsules are already approved for human use, and providing a number of "empty" capsules which do not contain pharmaceutically active ingredients may provide an alternative means of suppressing appetite by reducing the available stomach volume.

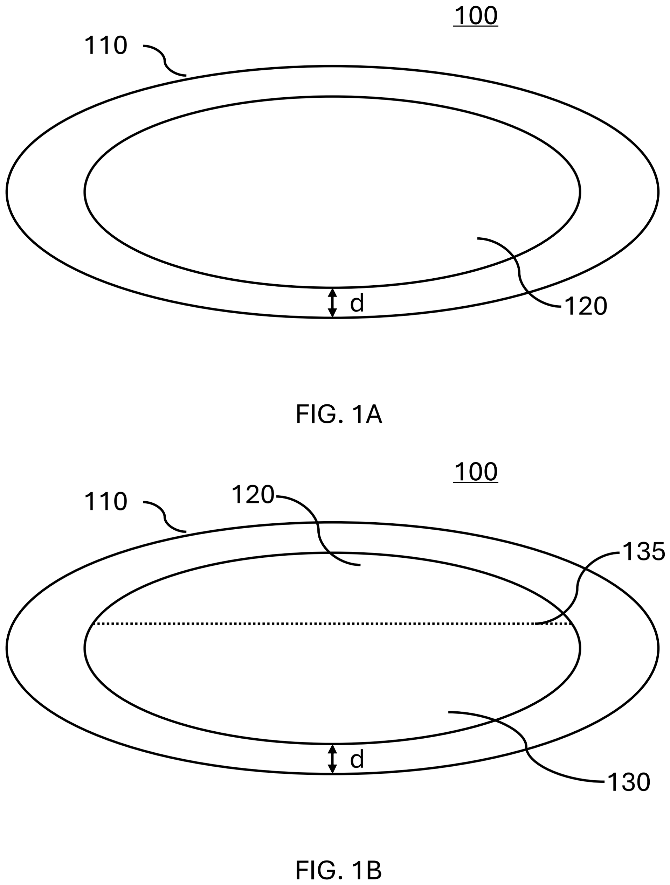

Fig. 1A shows an example structure of a capsule 100, according to some embodiments of the invention. Capsule 100 comprises an outer layer 110 and an inner volume 120 enclosed by outer layer 110. Outer layer 110 is adapted to persist in an acidic stomach environment, ensuring that capsule 100 maintains its integrity and functionality within the stomach for a prolonged period.

Persistence of capsule 100 contributes to the capsule's role in reducing stomach volume and suppressing appetite without the need for invasive procedures or pharmacological interventions. The persistence of capsule 100 may be configurable based on the selected outer layer material. For example, capsule 100 may be made of an outer layer material that takes more than 1 hour to dissolve, more than 5 hours to dissolve, or more than 24 hours to dissolve in the stomach environment.

In some embodiments, outer layer material 110 may be indissoluble in the stomach environment. It may take 2-5 days for such indissoluble capsules to pass through the digestive system and be removed in stool. The feeling of fullness and satiety, and thus the appetite supressing effect, may continue even when in the intestines, and may not be limited to the stomach.

Outer layer 110 may have a thickness d. Thickness d may differ at different points of capsule 100. In some embodiments, for example when capsule 100 is of a uniform shape such as a sphere, thickness d may be constant at all points of outer layer 110. Thickness d may be selected to contribute to the persistence of capsule 100 in the stomach environment. In the industry, capsule thickness is typically measured in non-SI units of thousandths of an inch (mils). A typical softgel capsule may have a wall thickness in the range 10-20 mils (e.g. 254-508 µm). For hard shell capsules a range of 100-110 µm may be typical.

Capsule 100 may be designed to be swallowed (e.g. taken orally) in its entirety, with a plurality of other capsules. A sufficient number of capsules 100 to achieve a noticeable stomach volume reducing effect may depend on the size of capsule 100. Capsule 100 may have a size which conforms with typical sizing standards used in the pharmaceutical industry. For example capsule 100 may have a size such as in Table 1:

**Table 1**

| Size (#) | Approximate internal volume (ml) | Approximate length (mm) | Approximate external diameter (mm) |
|---|---|---|---|
| 5 | 0.13 | 11.0 | 4.9 |
| 4 | 0.20 | 14.0 | 5.3 |
| 3 | 0.27 | 16.0 | 5.8 |
| 2 | 0.37 | 18.0 | 6.4 |
| 1 | 0.48 | 19.0 | 7.0 |
| 0 | 0.67 | 22.0 | 7.7 |
| 0E | 0.70 | 23.0 | 7.7 |
| 00 | 0.95 | 23.0 | 9.0 |
| 000 | 1.36 | 26.0 | 10.0 |

In some embodiments capsules/pellets of size 0, 0E, 00, or 000 are preferred for their larger volume as compared to other sizes.

Sizes other than those presented in Table 1 may be used.

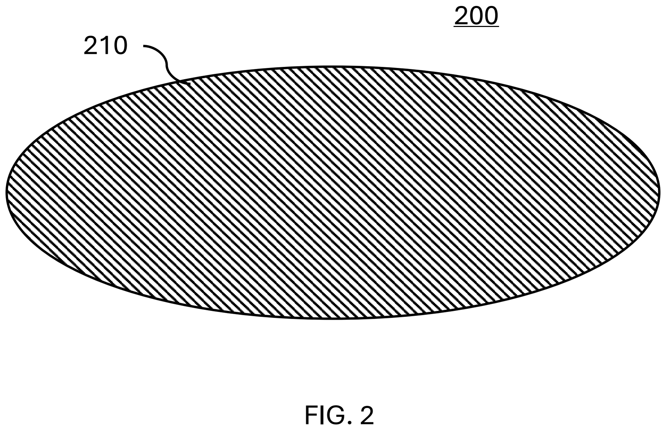

The inner volume 120 of the capsule 100 is enclosed (e.g. surrounded by) outer layer 110. Inner volume may be filled with various substances, as shown in Fig. 1B. It will be understood that filled may refer to including volumes less than a total volume of inner volume 120, e.g. fill does not necessarily imply completely filled or filled "to the brim". Fill line 135 demonstrates a partial filling of volume 120 with substance 130 below fill line 135. Substance 130 may be water, a low-calorie liquid, or a low-calorie powder. Accordingly, inner volume 120 may comprise water, a low-calorie liquid, or a low-calorie powder. In some embodiments inner volume 120 may be comprised throughout of the same material as the outer layer 110. In other words capsule 100 may be made entirely of outer layer material 110 throughout its cross section, without substantial voids or hollows. This is demonstrated more clearly in Fig. 2, with reference to pellet 200 described herein.

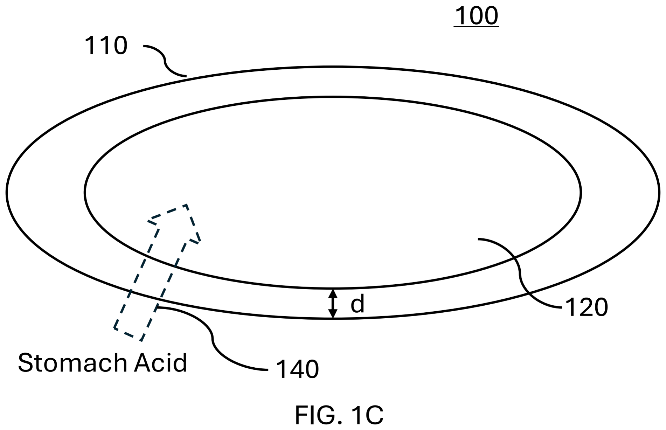

With reference to Fig. 1C, in some embodiments outer layer 110 may be selected (e.g. configured) to be semi-permeable to stomach acid, allowing for a controlled interaction between the stomach environment and the inner volume 120. The direction of permeability 140 indicates the selective passage of stomach acid into the inner volume 120. For example, outer layer 110 may comprise a membrane which is semi-permeable towards inner volume 120, as shown by arrow 140. Semi-permeable to stomach acid will be understood as permitting molecules of stomach acid through (e.g. diffusing across the membrane from the high concentration area of the stomach to the low concentration area of the inner volume), but preventing the passage of other, for example, large molecules. This directional permeability is achieved through the semi-permeable nature of the outer layer 110, which allows stomach acid to enter the inner volume 120 while preventing the escape of the capsule's contents. This interaction with stomach acid can lead to various effects, such as the expansion of the inner volume 120. This semi-permeability may allow capsule 100 to modulate the stomach's acidity and digestion process, for example by drawing an amount of stomach acid into the capsule, thereby reducing an amount of available stomach acid, and thereby slowing digestion of food consumed alongside, or consumed shortly after (e.g. within 1 hour), swallowing capsule 100. The slower rate of digestion may further contribute to the appetite suppressing effect of the capsule, such that fewer calories are consumed in a given time, and thus leading to natural weight management.

In some embodiments, the inner volume may include a gel or other material that expands when in contact with stomach acid, thereby increasing the occupying volume of the capsule within the stomach.

In some embodiments, the outer layer of the capsule is biodegradable. For example it may be designed, configured, and/or selected to be biodegradable, ensuring that the capsule can safely break down within the gastrointestinal environment after fulfilling its function of appetite suppression. This biodegradability is achieved through the selection of materials that are both biocompatible and capable of decomposing naturally within the body, such as polylactic acid (PLA), polyglycolic acid (PGA), or poly(lactic-co-glycolic acid) (PLGA). These materials are commonly used in the pharmaceutical industry for their ability to degrade into non-toxic byproducts that can be easily absorbed or excreted by the body. The biodegradable nature of the outer layer not only contributes to the environmental sustainability of the product but also reduce the risk of any potential adverse accumulation effects associated with long-term use. This feature enhances the safety of the capsules, making them suitable for repeated consumption as part of a weight management regimen.

Fig. 2 illustrates a pellet 200, which serves as an alternative embodiment of the described concept. Unlike the capsule embodiments, the pellet 200 lacks an inner volume and is composed entirely of the same material 210 throughout its structure. This design ensures that the pellet 200 is a solid entity, providing a consistent and uniform composition across its entire cross-section. For example, the material 210 extends throughout the cross-section of pellet 200, without substantial voids or empty spaces.

The material 210 of the pellet 200 is chosen for its ability to remain stable in an acidic stomach environment, similar to the materials used in slow-release drug delivery systems. This stability is important for maintaining the pellet's structural integrity and functionality within the stomach over an extended period, thereby prolonging the appetite-suppressing effect. Material 210 is selected to ensure biocompatibility and safety for consumption in large quantities, as the pellet is intended to be ingested in sufficient numbers to effectively reduce stomach volume and suppress appetite. Material 210 may be selected from the same materials used for outer layer 110 in capsule embodiments.

The solid nature of the pellet 200 offers a distinct advantage over capsules with an inner volume, as the pellet may take longer to dissolve in the stomach than a capsule having such a volume. This extended dissolution time enhances the duration of the appetite-suppressing effect, making the pellet 200 a viable option for individuals seeking a non-invasive and effective weight management solution. The consistent composition of material 210 throughout the pellet 200 also simplifies the manufacturing process, as there is no need to encapsulate an inner volume or manage the permeability of an outer layer.

Similar to capsule 100, pellet 200 interacts with the stomach environment by occupying space, thereby creating a sensation of fullness/satiety and helping individuals manage their appetite and reduce food intake. This spatial occupation is an important feature of the described technology, as it directly influences the reduction of food intake by limiting the stomach's capacity to accommodate additional food.

In some embodiments, a capsule/pellet may be designed to release an alkaline substance to neutralize stomach acid (or otherwise increase the pH), thereby reducing a total available amount of stomach acid in the stomach, slowing digestion, thereby contributing to the appetite suppressing effect and prolonging satiety. For example the capsule or pellet may be coated with an alkaline substance, such as calcium carbonate and/or magnesium carbonate. In some embodiments a capsule may include the alkaline substance inside the inner volume, released upon eventual breakdown of the capsule and thereby providing a lingering appetite suppressing effect (e.g. increasing duration by another hour) after the capsule is no longer occupying its original volume in the stomach.

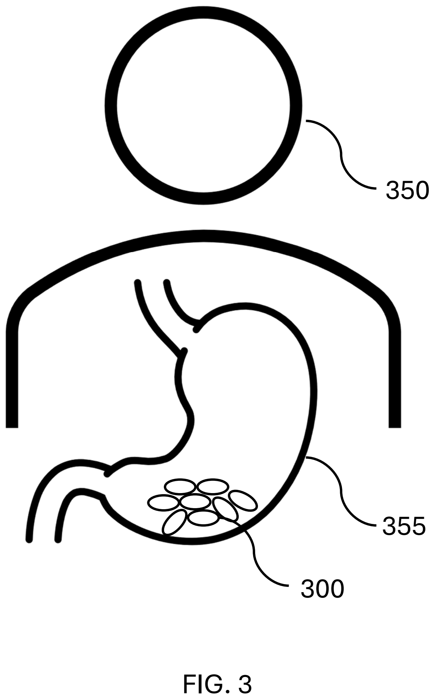

Fig. 3 shows a representation of capsules/pellets 300 occupying space within a stomach 355 of a user 350. As described, capsules/pellets according to embodiments of the invention are designed to be consumed in a quantity sufficient to effectively reduce the available volume within the stomach, thereby creating a sensation of fullness/satiety. This reduction in stomach volume is achieved without the need for invasive surgical procedures or pharmacological interventions.

A sufficient quantity of capsules/pellets to achieve an appetite suppressing effect may depend on a size of the capsules/pellets, but a typical number may include 5, capsules/pellets, 10 capsules/pellets, or more. According to some embodiments, the number may be determined based on a BMI (Body Mass Index) of the intended user.

According to some embodiments of the invention there is provided a kit comprising a plurality of capsules intended for appetite suppression, where each capsule includes an outer layer and an inner volume enclosed by the outer layer. For example the kit may include a plurality of capsules 100 as described herein. The kit may further include an indication that the kit is suitable for use in appetite suppression, which may, for example, be presented in the form of packaging, labeling, or instructions accompanying the kit. This indication may serve to inform users of the intended purpose of the capsules, ensuring they are used correctly to achieve the desired appetite-suppressing effect. The indication could comprise, for example, a label, packaging, a pamphlet, a leaflet, an instruction booklet, a website link, and/or QR (Quick Read) code.

In some embodiments, the kit may comprise a plurality of pellets for use in appetite suppression, each pellet formed of a material adapted to persist in an acidic stomach environment. The kit may comprise, for example, a plurality of pellets 200 as described herein.

In some embodiments the kit may comprise both capsules and pellets for the user to choose from. A kit may include capsules and/or pellets of different sizes, for examples capsules of a first size, and capsules of a second size.

The indication may include a recommended number of capsules/pellets to be taken. The recommended number may correspond to a quantity of capsules sufficient to induce the appetite suppressing effect, determined based on an occupying volume of each capsule in the stomach. Such a number may be referred to as an operative number. This number may be based on a BMI (Body Mass Index) of the user. For example a higher BMI may correlate to a higher number of capsules to be consumed.

Embodiments of the kit may be designed for ease of use, allowing individuals to incorporate the capsules into their daily routine without the need for medical supervision or prescription, thus providing a convenient and non-invasive solution for weight management. For example, capsules and/or pellets may be provided in vials or bottles as opposed to, for example, blister/punch packets, due to the number which are intended to be consumed and ease of access to the capsules and/or pellets. For example 10 capsules would not need to be individually punched out from a blister pack.

In some embodiments, the kit further includes means for filling the inner volume of the plurality of capsules. The means for filling may comprise various tools such as a syringe, injector, pump, pipette, or dropper, which facilitate the introduction of a substance into the inner volume of the capsules. This design allows users to customize the contents of the capsules according to their preferences or dietary needs, enhancing the versatility and user-friendliness of the kit. The ability to fill the capsules with different substances, such as water, low-calorie liquids, or powders, provides flexibility in achieving the desired appetite-suppressing effect while ensuring that the capsules remain safe and effective for consumption. The provided capsules may have a separable outer layer for this purpose, for example formed of two parts which can be connected, assembled, or otherwise joined together to contain the filling substance.

The kit may include a quantity of substance for filling the inner volume of the plurality of capsules. For example the kit may include a container, such as a bottle, pouch, sachet or packet of low-calorie liquid and/or powder for filling the capsules according to user preference.

According to some embodiments, the capsules/pellets are provided for use in a method of suppressing appetite. Such a method for the intended use of the capsules/pellets may comprise swallowing an operative number of the capsules/pellets. The capsules/pellets may be swallowed at the same time (or substantially the same time, e.g. one after the other within a space of less than 5 minutes). The operative number may be determined based on occupying volume of the capsules/pellets in the stomach.

For example, for a size 0 capsule, the operative number may be 15 capsules. For a size 0E capsule, the operative number may be 10 capsules. For a size 000 capsule, the operative number may be 5 capsules. In some embodiments, the operative number may be 3. It will be understood that the operative number may be a range, e.g. 3-5, 3-20, 5-20 capsules/pellets, which may be tailored based on factors such as user comfort, aggressiveness of weight loss regimen, body mass index, or the like. The operative number may be, for example, in the range 5-20 to ensure that the capsules collectively occupy sufficient space in the stomach to suppress appetite effectively. Accordingly it will be understood that the operative number may be described as greater than or equal to 5. In some embodiments the operative number may be greater than or equal to 3.

Whilst the materials described herein may be known substances or compositions known to one skilled in the art in the pharmaceutical context, their use in the form of a capsule or pellet independent of delivery of a pharmaceutically active ingredient is a further (e.g. second) alternative use which the inventor has realized can contribute to a safe means of appetite suppression when such capsules/pellets are taken in sufficient quantities.

Embodiments of the invention may be used for personal and private use. Embodiments of the invention may not require a prescription from a medical professional, because capsules/pellets according to some embodiments of the invention do not contain any pharmaceutically active ingredients.

Weight management may be achieved as a natural consequence of suppressing appetite by consuming fewer calories. In some embodiments, capsules or pellets according to embodiments of the invention may achieve an appetite suppressing effect in a mechanical way, rather than chemical or biological. For example, where the capsules or pellets contain no pharmaceutically active ingredients, the appetite suppressing effect may be achieved in a mechanical way by reducing volume in the stomach, thus eliciting a sensation of fullness (e.g. satiety) so that the user does not feel the need to consume as much food compared to without the use of the capsules/pellets, thus reducing calorie intake.

It should be recognized that embodiments of the invention may solve one or more of the objectives and/or challenges described in the background, and that embodiments of the invention need not meet every one of the above objectives and/or challenges to come within the scope of the present invention.

In the above description, an embodiment is an example or implementation of the inventions. The various appearances of "one embodiment," "an embodiment" or "some embodiments" do not necessarily all refer to the same embodiments.

Although various features of the invention may be described in the context of a single embodiment, the features may also be provided separately or in any suitable combination. Conversely, although the invention may be described herein in the context of separate embodiments for clarity, the invention may also be implemented in a single embodiment. While the invention has been described with respect to a limited number of embodiments, it should be understood by a person of ordinary skill in the art that one or more features from one particular embodiment or group of embodiments may be combined with features of another embodiment or group of embodiments.

Reference in the specification to "some embodiments", "an embodiment", "one embodiment" or "other embodiments" means that a particular feature, structure, or characteristic described in connection with the embodiments is included in at least some embodiments, but not necessarily all embodiments, of the inventions.

It is to be understood that the phraseology and terminology employed herein is not to be construed as limiting and are for descriptive purposes only.

It is to be understood that the details set forth herein do not construe a limitation to an application of the invention.

It is to be understood that the terms "including", "comprising", "consisting" and grammatical variants thereof do not preclude the addition of one or more components, features, steps, or integers or groups thereof and that the terms are to be construed as specifying components, features, steps, or integers.

If the specification or claims refer to "an additional" element, that does not preclude there being more than one of the additional elements.

It is to be understood that where the claims or specification refer to "a" or "an" element, such reference is not to be construed that there is only one of that element.

It is to be understood that where the specification states that a component, feature, structure, or characteristic "may", "might", "may" or "could" be included, that a particular component, feature, structure, or characteristic is not required to be included.

The description also includes the subject matter of the following clauses:
1. A capsule for use in appetite suppression, the capsule comprising an outer layer and an inner volume enclosed by the outer layer, wherein the outer layer is adapted to persist in an acidic stomach environment.
2. The capsule of clause 1, wherein the inner volume is empty and the capsule comprises no pharmaceutically active ingredients.
3. The capsule of clause 1, wherein the inner volume is comprised throughout of the same material as the outer layer, or wherein the inner volume comprises at least one of: water, a low-calorie liquid, a low-calorie powder, or an alkaline substance.
4. The capsule of clause 1, wherein the outer layer comprises a membrane selected to be semi-permeable to stomach acid towards the inner volume.
5. The capsule of clause 4, wherein the inner volume comprises a material that expands in volume when in contact with stomach acid.
6. The capsule of any of clauses 1-5, wherein the outer layer is biodegradable.
7. A kit comprising a plurality of capsules for use in appetite suppression, each capsule comprising an outer layer and an inner volume enclosed by the outer layer, wherein the outer layer is adapted to persist in an acidic stomach environment, the kit further comprising an indication that the kit is suitable for use in appetite suppression.
8. The kit of clause 7, comprising means for filling the inner volume of the plurality of capsules.
9. The kit of clause 8, further comprising a quantity of substance for filling the inner volume of the plurality of capsules.
10. The kit of clause 8, wherein the substance is one of: water, a low-calorie liquid, a low-calorie powder, or an alkaline substance.
11. The kit of any of clauses 7-10, wherein the indication comprises an operative number of capsules to be swallowed, the operative number determined based on an occupying volume of each capsule in the stomach.
12. The kit of any of clauses 7-11, wherein the operative number is greater than or equal to 5.
13. A capsule for use in a method of suppressing appetite, the capsule comprising an outer layer and an inner volume enclosed by the outer layer, wherein the outer layer is adapted to persist in an acidic stomach environment, and wherein the method of suppressing appetite comprises swallowing an operative number of the capsules, wherein the operative number is determined based on an occupying volume of each capsule in the stomach.
14. The capsule of clause 13, wherein the inner volume is empty and the capsule comprises no pharmaceutically active ingredients.
15. The capsule of clause 13, wherein the inner volume is comprised throughout of the same material as the outer layer, or wherein the inner volume comprises at least one of: water, a low-calorie liquid, a low-calorie powder, or an alkaline substance.
16. The capsule of clause 13, wherein the outer layer comprises a membrane selected to be semi-permeable to stomach acid towards the inner volume.
17. The capsule of any of clauses 13-16, wherein the outer layer is biodegradable.
18. The capsule of any of clauses 13-17, wherein the operative number is greater than or equal to 5.
19. A pellet for use in appetite suppression, the pellet formed throughout of a material adapted to persist in an acidic stomach environment.
20. A kit comprising a plurality of pellets for use in appetite suppression, each pellet formed of a material adapted to persist in an acidic stomach environment, the kit further comprising an indication that the kit is suitable for use in appetite suppression.
21. The kit of clause 20, wherein the indication comprises an operative number of pellets to be swallowed, the operative number determined based on an occupying volume of each pellet in the stomach.
22. A pellet for use in a method of suppressing appetite, the pellet formed throughout of a material adapted to persist in an acidic stomach environment, and wherein the method of suppressing appetite comprises swallowing an operative number of the pellets, wherein the operative number is determined based on an occupying volume of each pellet in the stomach.
23. The pellet of clause 19 or 22, wherein the material is biodegradable.
24. The pellet of any of clauses 19, 22, or 23, wherein the pellet is coated in an alkaline substance.
25. The pellet of any of clauses 22-24, wherein the operative number is greater than or equal to 5.

## Claims

1. A capsule for use in appetite suppression, the capsule comprising an outer layer and an inner volume enclosed by the outer layer, wherein the outer layer is adapted to persist in an acidic stomach environment.

2. The capsule of claim 1, wherein the inner volume is empty and the capsule comprises no pharmaceutically active ingredients.

3. The capsule of claim 1, wherein the inner volume is comprised throughout of the same material as the outer layer, or wherein the inner volume comprises at least one of: water, a low-calorie liquid, a low-calorie powder, or an alkaline substance.

4. The capsule of claim 1, wherein the outer layer comprises a membrane selected to be semi-permeable to stomach acid towards the inner volume.

5. The capsule of claim 4, wherein the inner volume comprises a material that expands in volume when in contact with stomach acid.

6. The capsule of any of claims 1-5, wherein the outer layer is biodegradable.

7. A kit comprising a plurality of capsules for use in appetite suppression, each capsule comprising an outer layer and an inner volume enclosed by the outer layer,
wherein the outer layer is adapted to persist in an acidic stomach environment,
the kit further comprising an indication that the kit is suitable for use in appetite suppression.

8. The kit of claim 7, comprising means for filling the inner volume of the plurality of capsules.

9. The kit of claim 8, further comprising a quantity of substance for filling the inner volume of the plurality of capsules.

10. The kit of claim 8, wherein the substance is one of: water, a low-calorie liquid, a low-calorie powder, or an alkaline substance.

11. The kit of any of claims 7-10, wherein the indication comprises an operative number of capsules to be swallowed, the operative number determined based on an occupying volume of each capsule in the stomach, optionally wherein the operative number is greater than or equal to 5.

12. A capsule for use in a method of suppressing appetite, the capsule comprising an outer layer and an inner volume enclosed by the outer layer, wherein the outer layer is adapted to persist in an acidic stomach environment, and wherein the method of suppressing appetite comprises swallowing an operative number of the capsules, wherein the operative number is determined based on an occupying volume of each capsule in the stomach.

13. A pellet for use in appetite suppression, the pellet formed throughout of a material adapted to persist in an acidic stomach environment.

14. A kit comprising a plurality of pellets for use in appetite suppression, each pellet formed of a material adapted to persist in an acidic stomach environment,
the kit further comprising an indication that the kit is suitable for use in appetite suppression.

15. A pellet for use in a method of suppressing appetite, the pellet formed throughout of a material adapted to persist in an acidic stomach environment, and wherein the method of suppressing appetite comprises swallowing an operative number of the pellets, wherein the operative number is determined based on an occupying volume of each pellet in the stomach.
